# EUROPEAN PATENT SPECIFICATION

(11) **EP 1 999 160 B1**
(45) Date of publication and mention of the grant of the patent: **30.12.2009**
(21) Application number: 07758243.5
(22) Date of filing: 09.03.2007
(51) Int. Cl.: C08B 37/08

(54) **ACRYLATED HYALURONIC ACID**
ACRYLIERTE HYALURONSÄURE
ACIDE HYALURONIQUE ACRYLE

(30) Priority: 14.03.2006 US 782581 P
(43) Date of publication of application: 10.12.2008
(73) Proprietor: Novozymes Biopharma DK A/S, 2880 Bagsvaerd (DK)
(72) Inventor: GROSS, Richard A., Plainview, New York 11803 (US); KUMAR, Vineet, West Wood, New Jersey 07675 (US)
(74) Representative: Lindum, Peter Würtz
(86) International application number: PCT/US2007/063671
(87) International publication number: WO 2007/106738

(56) References cited:
- WO-A-00/78988
- US-A1- 2002 192 182
- US-A1- 2005 129 734
- BURDICK JASON A ET AL: "CONTROLLED DEGRADATION AND MECHANICAL BEHAVIOR OF PHOTOPOLYMERIZED HYALURONIC ACID NETWORKS" BIOMACROMOLECULES, ACS, WASHINGTON, DC, US, vol. 6, 2005, pages 386-391, XP008069736 ISSN: 1525-7797 cited in the application
- SMEDS K A ET AL: "PHOTOCROSSLINKABLE POLYSACCHARIDES FOR IN SITU HYDROGEL FORMATION" JOURNAL OF BIOMEDICAL MATERIALS RESEARCH, WILEY, NEW YORK, NY, US, vol. 54, no. 1, January 2001 (2001-01), pages 115-121, XP008018719 ISSN: 0021-9304 cited in the application

## Description

### FIELD OF THE INVENTION

The present invention relates to a process for producing an acrylated hyaluronic acid (HA).

### BACKGROUND OF THE INVENTION

Hyaluronic acid (HA) is a natural and linear carbohydrate polymer belonging to the class of non-sulfated glycosaminoglycans. It is composed of beta-1,3-*N*-acetyl glucosamine and beta-1,4-glucuronic acid repeating disaccharide units with a molecular weight (MW) up to 6 MDa. HA is present in hyaline cartilage, synovial joint fluid, and skin tissue, both dermis and epidermis. HA may be extracted from natural tissues including the connective tissue of vertebrates, from the human umbilical cord and from cocks' combs. However, it is preferred today to prepare it by microbiological methods to minimize the potential risk of transferring infectious agents, and to increase product uniformity, quality and availability. (U.S. Patent No.6,951,743: WO 03/0175902).

Numerous roles of HA in the body have been identified. It plays an important role in biological organisms, as a mechanical support for cells of many tissues, such as skin, tendons, muscles and cartilage. HA is involved in key biological processes, such as the moistening of tissues, and lubrication. It is also suspected of having a role in numerous physiological functions, such as adhesion, development, cell motility, cancer, angiogenesis, and wound healing. Due to the unique physical and biological properties of HA (including viscoelasticity, biocompatibility, biodegradability), HA is employed in a wide range of current and developing applications within cosmetics, ophthalmology, rheumatology, drug and gene delivery, wound healing and tissue engineering. The use of HA in some of these applications is limited by the fact that HA is soluble in water at room temperature, i.e., about 20°C, it is rapidly degraded by hyaluronidase in the body, and it is difficult to process into biomaterials. Cross-linking of HA has therefore been introduced in order to improve the physical and mechanical properties of HA and its *in vivo* residence time.

Acrylated HA may be used to carry out enzyme-mediated cross-linking and produce materials in the form of hydrogels. Cross-linked HA may be used in cosmetic, biomedical and pharmaceutical applications. By cross-linking HA it is possible to design a compound with specific properties for specific applications. Acrylated HA provides functionalized reactive groups which can be further modified by a Michael-type addition to introduce bioactive peptides for different applications.

Acrylate groups on HA have been introduced by synthesis of glycidyl methacrylate-HA (GMHA) conjugates. GMHA derivatives are useful to prepare HA biomimetic hydrogels to promote tissue repair and wound healing. It has also been found that increased amounts of conjugated methacrylate groups correspond with increased cross-linked densities and decreased degradation rates, and have an insignificant effect on human aortic endothelial cell cytocompatability and proliferation. GMHA derivatives have been prepared by treating a 1% w/v solution of fermentation derived HA (∼2X10⁶ molecular weight) in distilled water with a 6-, 10- and 20-fold molar excess of glycidyl methacrylate in the presence of excess trimethyl amine and tetrabutyl ammonium bromide overnight at room temperature, followed by a 1 h incubation at 60°C. For example GMHA with a six-fold molar excess of GM was prepared by dissolution of 1.0 g HA in 100 ml distilled water. To the solution 2.2 ml triethyl amine, 2.2 ml glycidyl methacrylate and 2.2 g tetrabutyl ammonium bromide were added and stirred overnight followed by incubation at 60°C for an hour. By this process 5% methacrylation was achieved. Using 10- and 20-fold molar excess 7 and 11% acrylation was achieved (Leach and Schmidt, 2005, Biomaterials 26, 125-135; Yonese et al., 2002, Chem. Pharm. Bull. 50, 1341-1348; Schmidt et al., 2003, Biotechnology and Bioengineering 82(5), 578-589).

GMHA conjugates have also been prepared by dissolution of HA in carbonate buffer at pH 11.0. Glycidyl methacrylate was added to the above solution and the mixture stirred at room temperature for 7 days. The pH of the resulting solution was adjusted to 7.0 with HCl. It was filtered and freeze dried (Yonese, 2001. Journal of Controlled Release 73, 173-181). The GMHA conjugate was further cross-linked with hydroxyethyl acrylate to prepare a hydrogel.

Acrylate groups on HA have also been introduced by synthesis of *N*-3-aminopropyl methacrylamide-HA conjugates by modification of the carboxyl groups present in HA. Both native and enzymatically degraded HA were subsequently used for chemical modification. Both HAs were modified by using *N*-(3-aminopropyl) methacrylamide as an acrylating agent. The aminopropyl group reacted with a carboxyl group of HA in the presence of N-(3-dimethylaminopropyl)-N'-ethylcarbodiimide (EDCI) as a coupling agent resulting in formation of amide bond with pendant acryl groups at the other end. In a typical reaction HA was dissolved in distilled water. To the solution EDCI followed by *N*-3-aminopropyl methacrylamide were added. The reaction mixture was incubated at pH 6.5 for two hours followed by subsequent addition of EDCI and *N*-3-aminopropyl methacrylamide and further incubated for 2 hours at room temperature. The solution was filtered, dialyzed against NaCl followed by water and finally lyophilized to give methacrylated HA. The degree of acrylation achieved was roughly 10% (Tirelli et al., 2003, Biomaterials, 24, 893-900; Kim and Park 2002, Journal of Controlled Release, 80, 69-77).

Methacrylated hyaluronic acid (HAMA) has been synthesized by addition of methacrylic anhydride (∼20 fold) to a solution of 1% HA in deionized water adjusted to a pH of 8 with NaOH and reacted on ice for 24 hours. The modified polysaccharide was precipitated and washed with ethanol to remove methacrylic anhydride. In the process methacrylation up to 17% could be achieved. The low % methacrylation (17%) achieved was due to low reactivity of methacrylic anhydride with HA hydroxyls (Smeds and Grinstaff 2001, Journal of Biomedical Materials Research, 54, 115-121; Langer et al., 2005, Biomacmmotecules, 6, 386-391).

Known methods for acrylation of HA are time consuming and complicated, and there is a need in the art for a simpler process for acrylation of HA. It is an object of the present invention to provide a simple process for acrylation of hyaluronic acid.

### SUMMARY OF THE INVENTION

The present invention relates to methods of producing an acrylated hyaluronic acid, said method comprising the steps of:
(a) preparing an aqueous liquid with a pH of 7 to 11 comprising hyaluronic acid;
(b) preparing an organic liquid comprising acryl chloride and methylene chloride/diethyl ether; and
(c) mixing the organic liquid of (b) with the aqueous liquid of (a), wherein the pH is maintained between 7 and 11.

The processes of the present invention are very rapid due to very high reactivity of the acrylating reagent used, thereby reducing conventional reaction times. The % acrylation achieved is much higher using the processes of the present invention. Using the simple and rapid process a >90% acrylation can be achieved in a much shorter reaction time (1-2 hours) as compared to 17% reported to date by using complex processes. This is the highest % acrylation achieved without using any coupling agent. Moreover the side products obtained in the processes of the present invention are much less and could be easily removed as compared to reported protocols.

### DETAILED DESCRIPTION OF THE INVENTION

The present invention relates to processes of producing an acrylated hyaluronic acid comprising the following steps: a) preparing an aqueous liquid comprising hyaluronic acid wherein the pH is kept between 7 and 11: b) preparing of an organic liquid comprising acryloyl chloride and an organic solvent; and c) mixing the organic liquid of (b) and the aqueous liquid of (a) wherein the pH is maintained between 7 and 11.

Under the methods of the present invention, HA can be controllably acrylated and cross-linked to form highly hydrated and degradable hydrogels with a wide range of properties for different applications in wound healing, tissue engineering scaffold and a wide range of other biomedical applications.

The structural formula of the sodium salt of HA is shown below:

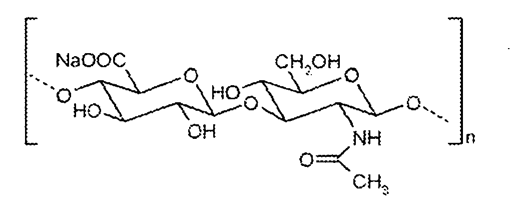

"Hyaluronic acid" is defined herein as an unsulphated glycosaminoglycan composed of repeating disaccharide units of *N*-acetylglucosamine (GlcNAc) and glucuronic acid (GlcUA) linked together by alternating beta-1,4 and beta-1,3 glycosidic bonds, which occur naturally in cell surfaces, in the basic extracellular substances of the connective tissue of vertebrates, in the synovial fluid of the joints, in the endobulbar fluid of the eye, in human umbilical cord tissue and in rooster combs. Hyaluronic acid is also known as hyaluronan, hyaluronate, or HA. The terms hyaluronan and hyaluronic acid are used interchangeably herein.

It is understood herein that the term "hyaluronic acid" encompasses a group of polysaccharides of *N*-acetyl-D-glucosamine and D-glucuronic acid with varying molecular weights or even degraded fractions of the same.

The present invention describes a simple process for acrylation of HA avoiding the use of phase transfer catalysts and coupling agents. A problem to be solved by the present invention is how to prepare acrylated hyaluronic acid intermediates, for the manufacturing of cross-linked HA based hydrogels, in a simple and rapid process.

The HA used in the present invention may be any available HA, including HA derived from natural tissues including the connective tissue of vertebrates, the human umbilical cord and from rooster combs. In a particular embodiment the hyaluronic acid or salt thereof is recombinantly produced, preferably by a Gram-positive bacterium or host cell, more preferably by a bacterium of the genus *Bacillus.* In another embodiment, the HA is obtained from a *Streptococcus* cell.

The host cell may be any *Bacillus* cell suitable for recombinant production of hyaluronic acid. The *Bacillus* host cell may be a wild-type *Bacillus* cell or a mutant thereof. *Bacillus* cells useful in the practice of the present invention include, but are not limited to, *Bacillus agaraderhens, Bacillus alkalophilus, Bacillus amyloliquefaciens, Bacillus brevis, Bacillus circulans, Bacillus clausii, Bacillus coagulans, Bacillus firmus, Bacillus lautus, Bacillus lentus, Bacillus licheniformis, Bacillus megaterium, Bacillus pumilus, Bacillus stearothermophilus, Bacillus subtilis,* and *Bacillus thuringiensis* cells. Mutant *Bacillus subtilis* cells particularly adapted for recombinant expression are described in WO 98/22598. Non-encapsulating *Bacillus* cells are particularly useful in the present invention.

In a preferred embodiment, the *Bacillus* host cell is a *Bacillus amyloliquefaciens, Bacillus clausii, Bacillus lentus, Bacillus licheniformis*, *Bacillus stearothermophilus* or *Bacillus subtilis* cell. In a more preferred embodiment, the *Bacillus* cell is a *Bacillus amyloliquefaciens* cell. In another more preferred embodiment, the *Bacillus* cell is a *Bacillus clausii* cell. In another more preferred embodiment, the *Bacillus* cell is a *Bacillus lentus* cell. In another more preferred embodiment, the *Bacillus* cell is a *Bacillus licheniformis* cell. In another more preferred embodiment, the *Bacillus* cell is a *Bacillus subtilis* cell. In a most preferred embodiment, the *Bacillus* host cell is *Bacillus subtilis* A164Δ5 (see U.S. Patent No. 5.891,701) or *Bacillus subtilis* 168Δ4.

The average molecular weight of the hyaluronic acid may be determined using standard methods in the art, such as those described by Ueno et al., 1988, Chem. Pharm. Bull. 36, 4971-4975; Wyatt, 1993, Anal. Chim. Acta 272, 1-40; and Wyatt Technologies, 1999, "Light Scattering University DAWN Course Manual" and "DAWN EOS Manual" Watt Technology Corporation, Santa Barbara. California.

In a preferred embodiment, the hyaluronic acid, or salt thereof, of the present invention has a molecular weight of about 10,000 to about 10,000,000 Da. In a more preferred embodiment it has a molecular weight of about 25,000 to about 5,000,000 Da. In a most preferred embodiment, the hyaluronic acid has a molecular weight of about 50,000 to about 3,000,000 Da.

In the processes of the present invention, the acryloyl chloride used may be any available acryloyl chloride.

Acryloyl chloride has the following structure: 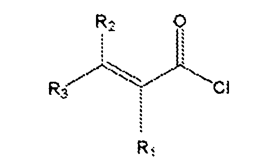 wherein, in a particular embodiment, R₁ is selected from the group consisting of hydrogen, methyl, chloride and COCl, R₂ is selected from the group consisting of hydrogen, methyl, phenyl, chloride, 2-chloro phenyl, COCl and CH₂COCl and R₃ is selected from the group consisting of hydrogen, methyl, chloride, 4-nitro phenyl, 3-trifluoromethylphenyl and styryl moieties.

Acryloyl chloride, also known as 2-propenoyl chloride or acrylic acid chloride is a clear, light yellow, flammable liquid with an acrid smell. It belongs to the acid chlorides group of compounds and is therefore a derivative of acrylic acid. This compound will give the common reactions of acid chlorides: it will react violently with water producing acrylic acid. Reactions with alcohols will result in the formation of esters and reactions with amines will generate amides. Acryloyl chloride is most commonly employed in organic synthesis for the introduction of acrylic moieties into other compounds. It is also used extensively in the preparation of acrylate monomers and polymers.

In a particular embodiment of the present invention the acryloyl chloride is selected from the group consisting of acryloyl chloride, methacryloyl chloride, crotonyl chloride, cinnamoyl chloride, fumaryl chloride, itaconyl chloride, 3,3-dimethylacryloyl chloride, trichloroacryloyl chloride, 2-chlorocinnamoyl chloride, trans-4-nitrocinnamoyl chloride, trans-3-(trifluoromethyl)cinnamoyl chloride, trans-3-(trifluoromethyl)cinnamoyl chloride and cinnamylidenemalonyl chloride.

In the methods of the present invention, HA is reacted with an acryloyl chloride according to the reaction shown below: 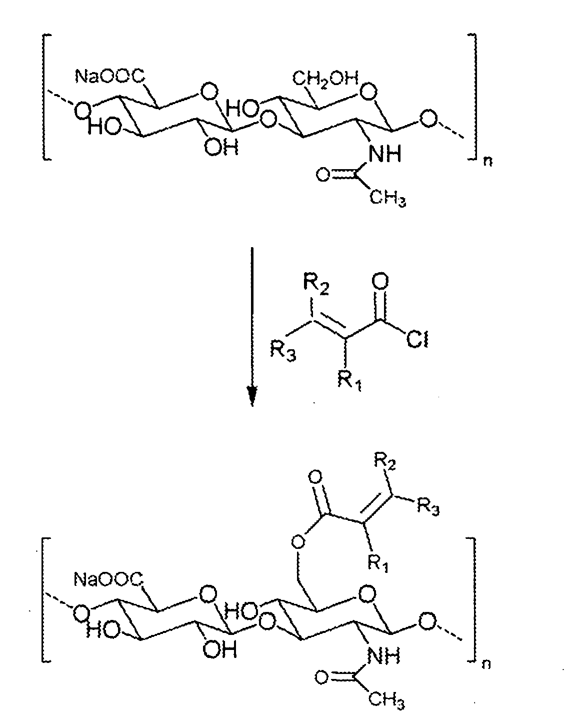 wherein R₁ is selected from the group consisting of hydrogen, methyl, chloride and COCl, and R₂ comprises a structure selected from the group consisting of hydrogen, methyl, phenyl, chloride, 2-chloro phenyl, COCl and CH₂COCl and R₃ is selected from the group consisting of hydrogen, methyl, chloride, 4-nitro phenyl, 3-trifluoromethylphenyl and styryl moieties.

In a particular embodiment of the present invention the aqueous liquid of a) is prepared in the following manner: HA is dissolved in water in particular deionised water to form an aqueous liquid comprising HA. Sodium hydroxide is added drop-wise to the aqueous liquid comprising HA. It is important that the hydroxide groups of HA are deprotonated. The aqueous liquid is left for a period of time at low temperatures to ensure the conversion of hydroxyl into hydroxide ions.

In a particular embodiment of the present invention the temperature of the aqueous liquid is lowered to around 0°C to 5°C after dissolution of the HA and is kept between 0°C and 15°C during the reaction. In a more particular embodiment of the present invention the temperature of the aqueous liquid is kept at 0°C and 5°C during the reaction.

In a particular embodiment of the present invention the pH is maintained between 7 and 11 during this first step of the process. In a more particular embodiment of the present invention the pH is kept between 8 and 10. In a most particular embodiment of the present invention the pH is kept between 8.5 and 9.5. The pH is maintained either by buffer and/or by addition of dilute sodium hydroxide.

The organic liquid is prepared by mixing acryloyl chloride and a low-boiling immiscible solvent. The low-boiling immiscible solvent may be selected from the group consisting of diethyl ether and dichloromethane.

Acrylation is mainly dependent on the pH of the solution. In a particular embodiment the acryloyl chloride is added drop-wise to the HA solution. Some acrylic acid will form whereby the pH goes down thus it is necessary to add a base to increase pH. In a particular embodiment the pH is maintained between 7 and 11 by using buffers and/or by addition of NaOH preferably 1 N-5 N NaOH. In a particular embodiment of the present invention the pH is kept between 7.5 and 10 during reaction. In a more particular embodiment of the present invention the pH is maintained between 7.5 and 9.5 during reaction. In a most particular invention the pH is maintained between 8 and 9.

The pH optimum for the present reaction is between 8 and 9.5. It may be difficult to maintain the pH stable during addition of acryloyl chloride as the pH changes are very fast due to the relatively rapid reaction of acryloyl chloride with water to form acrylic acid.

To be able to maintain the pH between 8 and 9.5 during the reaction of HA with acryloyl chloride it was found that the use of a syringe pump to add acryloyl chloride could solve the problem and keep addition of acryloyl chloride at 500-1000 uL/hour. By the use of a syringe pump a smaller amount of acryloyl chloride is added and thus the pH can be easily maintained. Thus in a particular embodiment a syringe pump is used to maintain the pH stable.

The ratio of HA to acryloyl chloride on a molar basis is preferably between 1:10 to 1:60. In a preferred embodiment, 50 mg of HA (0.125 mmol) in 25 ml of deionized water was treated with 120 microliters of acryloyl chloride (1.48 mmol) in a ratio of approximately 1:12, resulting in 17% acrylation of HA. In another preferred embodiment, the same concentration of HA (0.125 mmole) was treated with a higher amount of acryloyl chloride (250 microliters, 3.07 mmole) in a ratio of approximately 1:25, resulting in 34% acrylation of HA. In a more preferred embodiment, 0.125 mmol of HA was treated with 500 microliters of acryloyl chloride (6.15 mmol) in a ratio of approximately 1:50, resulting in 90% acrylation of HA.

After complete addition of the acryloyl chloride the liquid reaction mixture is stirred to ensure full reaction.

After the reaction is finished, the acrylated HA product is precipitated by addition of excess of an organic solvent like ethanol, acetone, methanol or isopropyl alcohol. For purification of the derivatized product, it is centrifuged, and washed with a solvent such as ethanol, methanol or acetone. The product may be dialyzed to provide a substantially pure acrylated HA product.

The acrylated HA may be formulated into a dry powder, e.g., by lyophilization or by spray drying.

The present invention also relates to acrylated HA having the following structure: 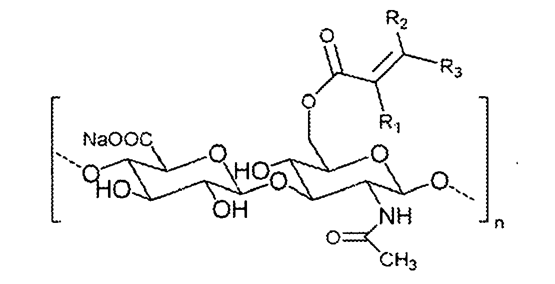 wherein R₁ is selected from the group consisting of hydrogen, methyl, chloride and COCl, and R₂ comprises a structure selected from the group consisting of hydrogen, methyl, phenyl, chloride, 2-chloro phenyl, COCl and CH₂COCl and R₃ is selected from the group consisting of hydrogen, methyl, chloride, 4-nitro phenyl, 3-trifluoromethylphenyl and styryl moieties.

In a particular embodiment, the present invention discloses an acrylated HA with the following structure:

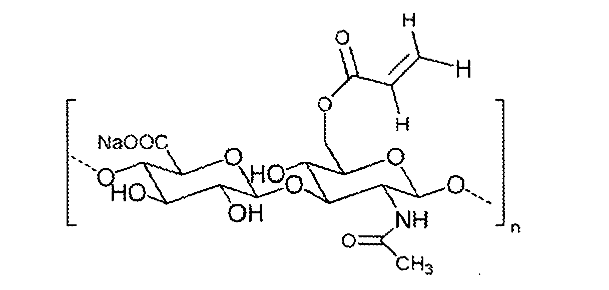

The acrylated HA products can be characterized by proton NMR. The % acrylation is determined from the integration values of the acrylate proton 5.66 ppm (1H) to the N-acetyl protons of hyaluronic acid (-NHCOCH₃, 3H, 2.0 ppm).

Acrylated HA may be used to carry out enzyme-mediated cross-linking and produce materials in the form of hydrogels.

Examples of enzyme-mediated cross-linking/polymerization:

Peroxidase (Horseradish) has been used as the enzyme catalyst to mediate the polymerization/cross-linking of the intramolecular or intermolecular acryl functional groups.

The enzyme classes of peroxidases and laccases are able to catalyze the above mentioned cross-linking reactions of the HA glycidyl acrylate and HA-acrylate.

The enzymatic cross-linking reaction of the chemically modified HA polymer is mild and environmentally friendly compared to alternative chemical methods.

Previously, the horseradish peroxidase (HRP) has been reported to mediate the free radical polymerization of methyl methacrylate (Kalra, B.; Gross, R.A.: Green Chemistry, 2002, 4, 174-178). The reaction can be carried out in water at ambient temperatures. As an example the HRP-mediated polymerization of sodium acrylate conducted in aqueous medium gave poly(sodium acrylate) in yields up to 88% within 24 h with Mw of approximately 119 KDa.

In a particular embodiment the enzyme is one of horseradish peroxidase, soybean peroxidase, and lignin peroxidase.

In a particular embodiment the enzyme is a recombinant enzyme.

In a particular embodiment the enzyme is a thermophilic enzyme.

In a particular embodiment the enzyme is a mesophilic enzyme.

In a particular embodiment of the present invention a method for polymerization! cross-linking of the acrylated hyaluronic acid product of the present invention comprising combining:
a) the acrylated hyaluronic acid product of claim 1;
b) a peroxide source;
c) an initiator;
d) an enzyme, and
e) a solvent.

A peroxide source useful in the present method may be any compound having an oxygen-oxygen bond, such as benzoyl peroxide, alkali and alkaline earth metal peroxides, mono- and dialkylperoxides, hydrogen peroxide bis-TMS ether, organic and inorganic peracids, or hydrogen peroxide. Reagents which generate a compound having an oxygen-oxygen bond under the reaction conditions are also peroxide sources as the term is used herein. Hydrogen peroxide is preferred because it generates only water as a by-product.

In a particular embodiment of the present invention the peroxide source may be hydrogen peroxide or alkyl peroxide.

In a particular embodiment of the present the acrylated hyaluronic acid product is one of methacrylate esters, acrylate esters, acrylamide, styrene, and acrylic acid and salts thereof.

In a particular embodiment of the present invention the initiator is a beta-dicarbonyl compound or beta-diketone.

In a particular embodiment the solvent is water or an organic solvents, or mixtures thereof.

In a particular embodiment the polymerization/cross-linking is done under an inert atmosphere.

### EXAMPLES

### Example 1

High molecular weight hyaluronic (700,000-1,000,000 Dalton, 50 mg) acid was dissolved in deionized water (25 ml). The temperature was reduced to 0°C.

To the above solution one drop of 0.33 N NaOH was added. The pH changed from 5.4 to 9.5. The solution was stirred at this pH for 30 minutes. A mixture of equal amount of acryloyl chloride (100-500 uL) and dichloromethane (100-500 ml) was prepared. It was added drop by drop over an hour to the hyaluronic acid reaction mixture. During addition of acryloyl chloride dropwise, the pH was maintained between 8-9 by dropwise addition of 1 N-5 N NaOH.

After complete addition of acryloyl chloride, the solution was allowed to stir for another one hour. Throughout the reaction low temperature (0-5°C) was maintained. It was filtered. The filtrate was precipitated using a large excess of cold ethanol (500 ml-1000 ml) and washed with ethanol. It was centrifuged, dialyzed and lyophilized.

### Example 2

High molecular weight hyaluronic acid (700,000-1,000,000 Dalton, 50 mg) was dissolved in 0.25-2.0 M phosphate buffer (25 ml) having pH 8.0. The temperature was reduced to 0°C. The solution was stirred at this pH for 30 minutes. A mixture of equal amount of acryloyl chloride (100-500 uL) and dichloromethane (100-500 ml) was added drop by drop over an hour to the hyaluronic acid reaction mixture. After complete addition of acryloyl chloride, the solution was allowed to stir for another one hour. Throughout the reaction low temperature (0-5°C) was maintained. The resulting product was filtered. The filtrate was precipitated using a large excess of cold ethanol (500 ml-1000 ml) and washed with ethanol. It was centrifuged, dialyzed and lyophilized.

### Example 3

High molecular weight hyaluronic acid (700,000-1,000,000 Dalton, 50 mg) was dissolved in deionized water (25 ml). The temperature was reduced to 0°C.

To the above solution one drop of 0.33 N NaOH was added. The pH changed from 5.4 to 9.5. The solution was stirred at this pH for 30 minutes. Acryloyl chloride (100-500 uL) was added dropwise using a syringe pump. The rate of addition of acryloyl chloride was 500-2000 uL/hr. The pH was maintained between 8-9 by dropwise addition of 1 N-5 N NaOH.

After complete addition of acryloyl chloride, the solution was allowed to stir for one hour. Throughout the reaction low temperature (0-5°C) was maintained. The reaction mixture was filtered. The filtrate was precipitated using a large excess of cold ethanol (500 ml-1000 ml) and washed with ethanol. It was centrifuged, dialyzed and lyophilized.

Using the above process different acrylated hyaluronic acid derivatives with varying percent acrylation were obtained by treatment with varying amounts of acryloyl chloride used for acrylation. The % acrylation was calculated by comparing the signal at 2.02 (3H, -NHCOCH₃) and 5.6 (1H, H_{A} proton of the acryl moiety introduced as shown below):

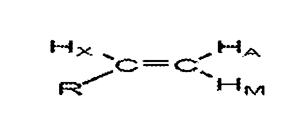

As an example 50 mg of HA (0.125 mmol) in 25 ml of deionized water on treatment with 120 microliters of acryloyl chloride (1.48 mmol) resulted in 17% acrylation. Using the same concentration of HA (0.125 mmol) and treatment with higher amount of acryloyl chloride (250 microliters, 3.07 mmol) resulted in 34% acrylation. To achieve higher acrylation, HA (0.125 mmol) was treated with 500 microliters of acryloyl chloride (6.15 mmol) which resulted in 90% acrylation of hyaluronic acid. This is the highest % acrylation achieved without using any coupling agent. Higher % acrylation is achieved due to high reactivity of acryloyl chloride with primary hydroxyls of HA. The yields of the modified products are >90%.

### Example 4

¹H NMR (Varian-300) was used to determine the final functionality and purity of the acrylated hyaluronic acid (in D₂O). The samples were in ²H₂O with the ²HOH peak at 4.79 ppm used as the reference line. Proton-NMR of the acrylated hyaluronic acid revealed distinct acryl peaks at (5.66 ppm, 1H and 6.04 ppm, 2H). The peak at 5.66 ppm appears as a doublet of doublet (11 Hz and 2 Hz). Due to cis coupling of H_{A} across the double bond to the Hx proton next to the carbonyl group, a coupling constant of (11 Hz) is obtained. It also couples geminally to the proton H_{M} and a coupling constant of 2 Hz is observed. H_{M} and H_{X} protons appear at 6.04 ppm. A coupling constant of 17 Hz and 13 Hz is observed due to coupling of H_{X} to H_{M} (trans-) across the double bond and coupling of H_{X} to H_{A} (cis-) across the double bond. Similarly HM proton couples (trans-) across the double bond to the H_{X} proton and a coupling constant of 17 Hz is observed. Since H_{M} and Hx protons appear in the same region (6.04 ppm) and due to low Δν/J ratio, it is not possible to resolve all the coupling constants. The degree of modification was determined from the relative integrations of the acrylate to N-acetyl protons of hyaluronic acid (-NHCOCH₃, 3H, 2.0 ppm).

## Claims

1. A process for the preparation of an acrylated hyaluronic acid product comprising the following steps:
a) preparing an aqueous liquid comprising hyaluronic acid wherein the pH is kept at a suitable pH;
b) preparing an organic liquid comprising acryloyl chloride and an organic solvent; and
c) mixing the organic liquid of (b) and the aqueous liquid of (a), wherein the pH is maintained at a suitable pH.

2. The process of claim 1, wherein the mixing in step c) is achieved by stirring.

3. The process of claim 1, wherein step a) is maintained at a pH between 7 and 11.

4. The process of claim 1, wherein the aqueous liquid of step a) comprises a phosphate buffer.

5. The process of claim 4, wherein the phosphate buffer is present in an amount between 0.1 molar to 1 molar.

6. The process of claim 1, wherein step c) is maintained at a pH between 7 and 11.

7. The process of claim 1, wherein step c) is maintained at a pH between 7 and 11 by use of a buffer and/or drop-wise addition of dilute NaOH.

8. The process of claim 1, wherein the temperature is kept between 0°C and 15°C during step a).

9. The process of claim 1, wherein the temperature is kept between 0°C and 5°C during step c).

10. The process of claim 1, wherein the pH of the aqueous liquid of step a) is adjusted by adding NaOH drop-wise.

11. The process of claim 1, wherein the pH of the aqueous liquid of step a) is adjusted by a buffer and/or by adding NaOH drop-wise.

12. The process of claim 1, wherein the addition of acryloyl chloride in step c) is achieved by using a syringe pump.

13. The process of claim 1, further comprising a recovery step.

14. The process of claim 13, wherein the recovery step comprises a precipitation step.

15. The process of claim 13, wherein the recovery step further comprises a filtration step of the reaction mixture and a precipitation of the filtrate.

## Patentansprüche

1. Verfahren für die Herstellung eines acrylierten Hyaluronsäureprodukts, umfassend die folgenden Schritte:
a) Herstellen einer Hyaluronsäure umfassenden wässrigen Flüssigkeit, wobei der pH bei einem geeigneten pH gehalten wird;
b) Herstellen einer organischen Flüssigkeit, umfassend Acryloylchlorid und ein organisches Lösungsmittel; und
c) Mischen der organischen Flüssigkeit von (b) und der wässrigen Flüssigkeit von (a), wobei der pH bei einem geeigneten pH beibehalten wird.

2. Verfahren nach Anspruch 1, wobei das Mischen in Schritt c) durch Rühren erreicht wird.

3. Verfahren nach Anspruch 1, wobei Schritt a) bei einem pH zwischen 7 und 11 beibehalten wird.

4. Verfahren nach Anspruch 1, wobei die wässrige Flüssigkeit von Schritt a) einen Phosphatpuffer umfasst.

5. Verfahren nach Anspruch 4, wobei der Phosphatpuffer in einer Menge zwischen 0,1 M bis 1 M vorliegt.

6. Verfahren nach Anspruch 1, wobei Schritt c) bei einem pH zwischen 7 und 11 beibehalten wird.

7. Verfahren nach Anspruch 1, wobei Schritt c) bei einem pH zwischen 7 und 11 durch Verwendung eines Puffers und/oder tropfenweise Zugabe von verdünnter NaOH beibehalten wird.

8. Verfahren nach Anspruch 1, wobei die Temperatur zwischen 0 °C und 15 °C während Schritt a) gehalten wird.

9. Verfahren nach Anspruch 1, wobei die Temperatur zwischen 0 °C und 5 °C während Schritt c) gehalten wird.

10. Verfahren nach Anspruch 1, wobei der pH der wässrigen Flüssigkeit von Schritt a) durch tropfenweises Hinzufügen von NaOH eingestellt wird.

11. Verfahren nach Anspruch 1, wobei der pH der wässrigen Flüssigkeit von Schritt a) durch einen Puffer und/oder durch tropfenweises Hinzufügen von NaOH eingestellt wird.

12. Verfahren nach Anspruch 1, wobei die Zugabe von Acryloylchlorid in Schritt c) unter Verwendung einer Spritzenpumpe erreicht wird.

13. Verfahren nach Anspruch 1, des Weiteren umfassend einen Gewinnungsschritt.

14. Verfahren nach Anspruch 13, wobei der Gewinnungsschritt einen Präzipitationsschritt umfasst.

15. Verfahren nach Anspruch 13, wobei der Gewinnungsschritt des Weiteren einen Filtrationsschritt des Reaktionsgemisches und eine Präzipitation des Filtrates umfasst.

## Revendications

1. Procédé de préparation d'un produit d'acide hyaluronique acrylé comprenant les étapes suivantes :
a) préparer un liquide aqueux comprenant de l'acide hyaluronique où le pH est conservé à un pH approprié ;
b) préparer un liquide organique comprenant du chlorure d'acryloyle et un solvant organique ; et
c) mélanger le liquide organique de b) et le liquide aqueux de a), où le pH est maintenu à un pH approprié.

2. Procédé selon la revendication 1, où le mélange de l'étape c) est effectué par agitation.

3. Procédé selon la revendication 1, où l'étape a) est maintenue à un pH entre 7 et 11.

4. Procédé selon la revendication 1, où le liquide aqueux de l'étape a) comprend un tampon phosphate.

5. Procédé selon la revendication 4, où le tampon phosphate est présent en une quantité entre 0,1 molaire et 1 molaire.

6. Procédé selon la revendication 1, où l'étape c) est maintenue à un pH entre 7 et 11.

7. Procédé selon la revendication 1, où l'étape c) est maintenue à un pH entre 7 et 11 par utilisation d'un tampon et/ou un ajout goutte à goutte de NaOH diluée.

8. Procédé selon la revendication 1, où la température est conservée entre 0°C et 15°C pendant l'étape a).

9. Procédé selon la revendication 1, où la température est conservée entre 0°C et 5°C pendant l'étape c).

10. Procédé selon la revendication 1, où le pH du liquide aqueux de l'étape a) est ajusté en ajoutant NaOH goutte à goutte.

11. Procédé selon la revendication 1, où le pH du liquide aqueux de l'étape a) est ajusté par un tampon et/ou en ajoutant NaOH goutte à goutte.

12. Procédé selon la revendication 1, où l'ajout de chlorure d'acryloyle à l'étape c) est réalisée en utilisant une pompe seringue.

13. Procédé selon la revendication 1, comprenant en outre une étape de récupération.

14. Procédé selon la revendication 13, où l'étape de récupération comprend une étape de précipitation.

15. Procédé selon la revendication 13, où l'étape de récupération comprend en outre une étape de filtration du milieu réactionnel et une précipitation du filtrat.
